# EUROPEAN PATENT SPECIFICATION

(11) **EP 1 795 162 B1**
(45) Date of publication and mention of the grant of the patent: **13.06.2012**
(21) Application number: 04788165.1
(22) Date of filing: 27.09.2004
(51) Int. Cl.: A61F 13/472, A61F 13/15

(54) **INDIVIDUALLY PACKAGED ABSORPTIVE ARTICLE AND METHOD OF MANUFACTURING THE SAME**
EINZELN VERPACKTER SAUGFÄHIGER ARTIKEL UND HERSTELLUNGSVERFAHREN DAFÜR
ARTICLE ABSORBEUR EMBALLE INDIVIDUELLEMENT ET PROCEDE DE FABRICATION DE CELUI-CI

(43) Date of publication of application: 13.06.2007
(73) Proprietor: DAIO PAPER CORPORATION, Shikokuchuo-shi, Ehime 799-0492 (JP)
(72) Inventor: YASUDA, Ai, c/o DAIO SANITARY PRODUCTS CO., LTD., Shizuoka 4190201 (JP); KUME, Yukio, c/o DAIO SANITARY PRODUCTS CO., LTD., Shizuoka 4190201 (JP); SHIRATORI, Kiminori, DAIO SANITARY PRODUCTS CO LTD, Shizuoka 4190201 (JP)
(74) Representative: Held, Stephan
(86) International application number: PCT/JP2004/014069
(87) International publication number: WO 2006/035480

(56) References cited:
- JP-B2- 7 121 725
- JP-Y2- 2 589 604
- US-A- 5 462 166
- US-A1- 2004 149 613

## Description

### [Field of the Invention]

The present invention relates to individually packaged absorbent articles and a method of manufacturing the same.

### [Prior Art

In the prior art, absorbent articles are sold in form of a package including several pieces thereof, and at use, they are taken out one by one. Especially with regard to sanitary napkins, from the viewpoint of hygiene, individual packaging where absorbent articles are packed one by one is carried out. (For example, refer to Patent Publication 1.)

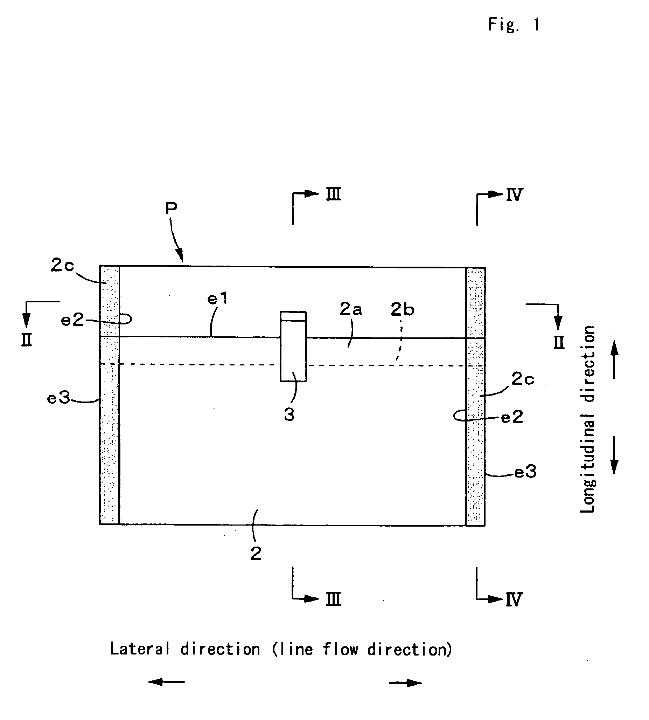

The mode of individually packaged absorbent articles according to the prior art is shown in FIG. 1 - FIG. 4. A package sheet 2 has a cylindrical shape where both end parts 2a, 2b thereof in the longitudinal direction orthogonal to a packaging line flow direction are overlapped, and the end edge e1 of the front surface side portion 2a of the overlapped portion is made the opening start end, and overlapped portions 2c, 2c of both the end parts in the lateral direction along the packaging line flow direction are sealed by pressure, and an absorbent article 1 is included in the center of the sheet 2. The pressure sealed portions 2c, 2c are formed so as to be substantially continuous from one end to the other end in the longitudinal direction. And, the end edges e2 at the center side in the lateral direction of the pressure sealed portions 2c, 2c are each formed in a straight line along the longitudinal direction. Further, end edges in the lateral direction of the package sheet (end edges at the outer sides in the lateral direction of the pressure sealed portions 2c, 2c) are each formed in a straight line along the longitudinal direction too.

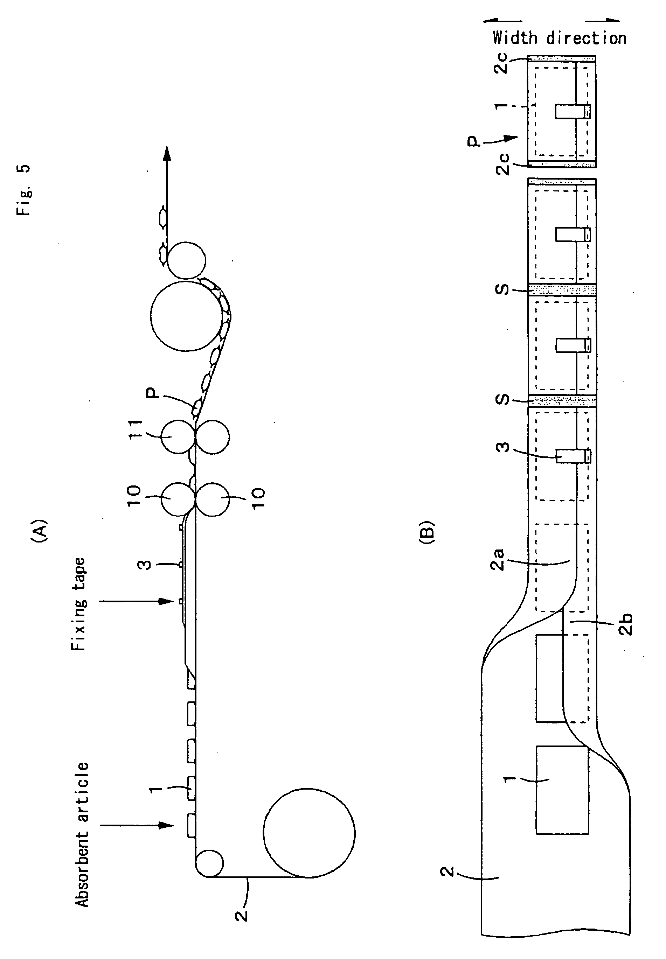

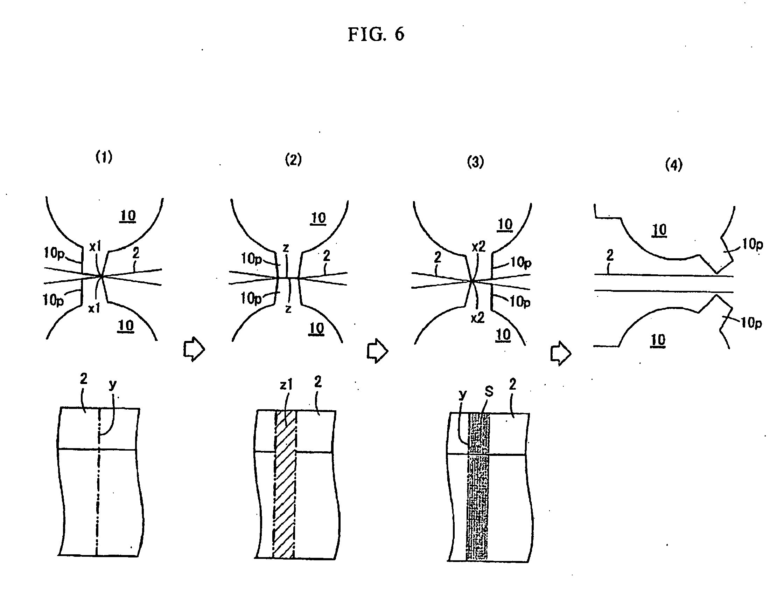

On the other hand, in such an individual package, in the prior art, for example a method shown in FIG. 5 has been generally employed (for example, refer to Patent Publication 2) . That is, first, a band shaped package sheet 2 is fed out to a packaging line, and absorbent articles 1 are placed on this band shaped package sheet 2L, and both end parts 2a, 2b in the width direction of the band shaped package sheet 2 are folded back to the center side, and thereby the absorbent articles 1 are packaged. Thereafter, the band shaped package sheet 2 containing the absorbent articles 1 is made to go through a pair of rolls 10, 10 each having a convex streak that substantially continues from one end to the other end in the width direction (refer to FIG. 6 described later herein), and sealed portions S, S preceding and following the portion containing each absorbent article 1 in the line flow direction are pressed between the convex streak of the roll 10 at one side and the convex streak of the roll 10 at the other side, and thereby pressure sealing that substantially continues from one end to the other end in the width direction is carried out. Thereafter, the middle in the line flow direction of the pressure sealed portions S, S is cut by cutting apparatus 11, and thereby absorbent articles P as individually packaged as explained above are obtained.

However, in the packaging mode in the prior art, end edges at the center side in the lateral direction of the pressure sealed portions are formed each in a straight line along the lateral direction, and accordingly, the end edges at the center side are cut over the entire longitudinal direction due to the pressure sealing at packaging, and the sealing has made no sense in many cases.
Patent Publication 1 : Japanese Utility Model Application Publication No.2589604
Patent Publication 2 : Japanese Patent Application Publication No.H07-121725
Patent Publication 3: United States Patent Application US 2004/149 615 A1
Patent Publication 4: United States Patent No. US 5,462,166A

### [Disclosure of the Invention]

### [Problems to be Solved by the Invention]

Accordingly, the main obj ect of the present invention is to prevent the cutting due to the pressure sealing at packaging.

### [Means to Solve the Problems]

The present inventors made close studies on the cutting of the pressure sealed portions, and have obtained the following knowledge. That is, in the case where pressure sealing is carried out between the convex streak 10p of a first roll 10 and the convex streak 10p of a second roll 10 that rotate in the line flow direction as shown in FIG. 6,
(1) edges x1 at the front side in the rotation direction of the convex streaks 10p first contact with the package sheet 2,
(2) next, the entire surfaces Z1 of the convex streaks 10p contact with it,
(3) thereafter, through the state where only edges x2 at the rear side in the rotation direction of the convex streaks 10p contact with the package sheet 2,
(4) the convex streaks 10p leave the package sheet 2.

In such a state transition, in the sates of (1) and (3), the pressure by the preceding and following edges x1, x2 of the convex streaks 10p, 10p works onto a small area. Further, this pressure works in a line shape y that substantially continues in the longitudinal direction orthogonal to the line flow direction. Accordingly, the package sheet 2 is apt to be cut off.

The present invention has been made on the basis of such knowledge, and is characterized by that a mode is adopted where the pressure sealed portion substantially continues from one end to the other end in the longitudinal direction, and the end edges at the center side in the lateral direction of the pressure sealed portion is not formed into a straight line along the longitudinal direction.

That is, in order to achieve the above object, according to one aspect of the present invention, there is provided an individually packaged absorbent article comprising an absorbent article and a package sheet to pack the absorbent article, wherein
the package sheet is formed in a cylindrical shape such that both end parts of the package sheet in a longitudinal direction are overlapped with each other, and an end edge of a front surface side portion of an overlapped portion serves as an opening start end, and overlapped portions of both end parts in a lateral direction are sealed by pressure, and wherein
the pressure sealed portions are formed so as to substantially continue from one end to the other end in the longitudinal direction, and end edges of the pressure sealed portions on lateral center side are not formed to be straight along the longitudinal direction.

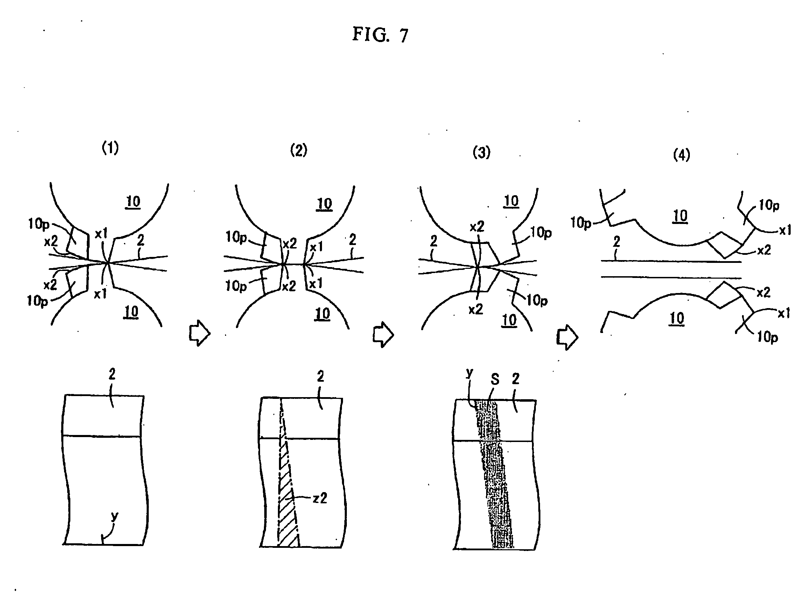

In the case when such pressure sealing is carried out, for example as shown in FIG. 7, the preceding and following edges x1, x2 of the convex streaks 10p press at the same time only partially in the width direction in the package sheet 2. In other words, in the states of (1) and (3), the preceding and following edges x1, x2 of the convex streaks 10p and the contact portion y of the package sheet 2 do not substantially continue in the longitudinal direction. Accordingly, even if cutting by the preceding and following edges x1, x2 of the convex streaks 10p occurs, it occurs only in a part in the longitudinal direction of the package sheet 2, and does not lead to cutting in the entire longitudinal direction. Since the preceding and following edges x1, x2 of the convex streaks 10p finally contact with the entire length of the package sheet 2 in the longitudinal direction by the rotation of the rolls 10, seemingly it looks like that cutting progresses in sequence accordingly, and the entire length of the package sheet 2 in the longitudinal direction is cut. However, as shown in FIG. 7, such a state does not occur in practice. For example, now suppose a state where the preceding edges x1 of the convex streaks 10p of the rolls 10 start contacting with the package sheet 2. In the convex streaks 10p of the rolls 10, the portions that first contact with the package sheet 2 in the roll width direction first shift to the surface contact state (same as the above state (2) except that there is a difference whether part or the whole of the surface in the roll width direction). Therefore, not only the preceding edges x1 of the convex streaks 10p that contact with the package sheet 2 with delay contact with the package sheet 2, but the range z2 including the portions that first start contacting and shift to the surface contact state contacts with the package sheet 2. When the following edges x2 of the convex streaks 10p leave the package sheet 2, the above contact state is reversed. Accordingly, even if there occurs cutting by the preceding and following edges x1, x2 of the convex streaks 10p, it occurs only in a part in the longitudinal direction of the package sheet 2. Accordingly, it is possible to prevent a situation where the entire end edges at the center side of the lateral direction of the pressure sealed portion 2c are cut and a sealing failure occurs.

In the present invention, as a mode where "end edges of the pressure sealed portions on lateral center side are not formed to be straight along the longitudinal direction ", the mode where the end edges of the pressure sealed portions on the lateral center side thereof are formed into a wave shape. In particular, the mode of a wave shape has an advantage that the showing such as functional beauty and premium accents, and lovely looks (extremely important function in sanitary napkins and the like) become preferable. Needless to say, the "curved shape" in the present invention does not include a straight line.

In the present invention, it is preferable that the end edges in the lateral direction of the package sheet are formed in parallel so as to laterally correspond to the end edges of the pressure sealed portions on the lateral center side thereof. In such a mode, the pressure sealed portion shows a geometric shape with a sense of unity, and the showing such as functional beauty and premium accents, and lovely looks become remarkably preferable, and the pressure sealed portion extends in the same width along the end edges in the lateral direction of the package sheet, and accordingly, the detachment resistance of the pressure sealed portion at opening the package becomes stable, and it is possible to open the package smoothly.

In the present invention, it is preferable that a part or whole on the opening direction side of the end edges of the pressure sealed portions on the lateral center side thereof extends from the opening start end in the opening direction so as to be away with a distance from the lateral center side. In a mode that satisfies this requirement, the direction of the force that works onto the pressure sealed portion at least at early stage of opening start becomes close to the extending direction of the end edges of the pressure sealed portion, and consequently, it is possible to open the package smoothly.

On the other hand, according to another aspect of the present invention, there is provided a method of manufacturing an individually packaged absorbent article, comprising steps of
feeding a band shaped package sheet to a packaging line;
placing absorbent articles on the band shaped package sheet;
thereafter, folding back an end part on one side and an end part on the other side in a width direction of the band shaped package sheet and thereby packing the absorbent articles;
passing the band shaped package sheet containing the absorbent articles between a pair of rolls each having a convex streak that substantially continues from one end to the other end in the width direction, and pressing a portion to be sealed preceding and a portion to be sealed following in a flow direction with respect to a part of the band shaped package sheet which contains the absorbent article between the convex streak of the roll at one side and the convex streak of the roll at the other side, and thereby carrying out a pressure sealing that substantially continues from one end to the other end in the width direction, and
thereafter, cutting each of the pressure sealed portions at its middle in the line flow direction, and thereby obtaining individually packaged absorbent articles; wherein
the pressure sealing that substantially continues from one end to the other end in the width direction of the package sheet is carried out such that each of a preceding edge and a following edge of the convex streak in a rotation direction presses at a time only a part of an entire length from one end to the other end in the width direction.

When the pressure sealing is carried out so that the respective preceding and following edges in the rotation direction in the convex streaks press only part from one end to the other end in the width direction of the package sheet at the same time, and the pressure sealing that substantially continues from one end to the other end in the width direction is carried out in this manner, as described previously, even if cutting by the preceding and following edges of the convex streaks occurs, it occurs only in the part in the longitudinal direction of the package sheet, and does not lead to cutting in the whole of the longitudinal direction. Accordingly, it is possible to prevent such a situation where the entire end edges at the center side of the lateral direction of the pressure sealed portion are cut and a sealing failure occurs.

### [Effects of the Invention]

As explained heretofore, according to the present invention, it is possible to obtain various advantages, such as the prevention of cutting by pressure sealing at packaging and so forth.

### [Best Mode for Carrying out the Invention]

### <Preferred Embodiment concerning Package Structure of Absorbent Articles>

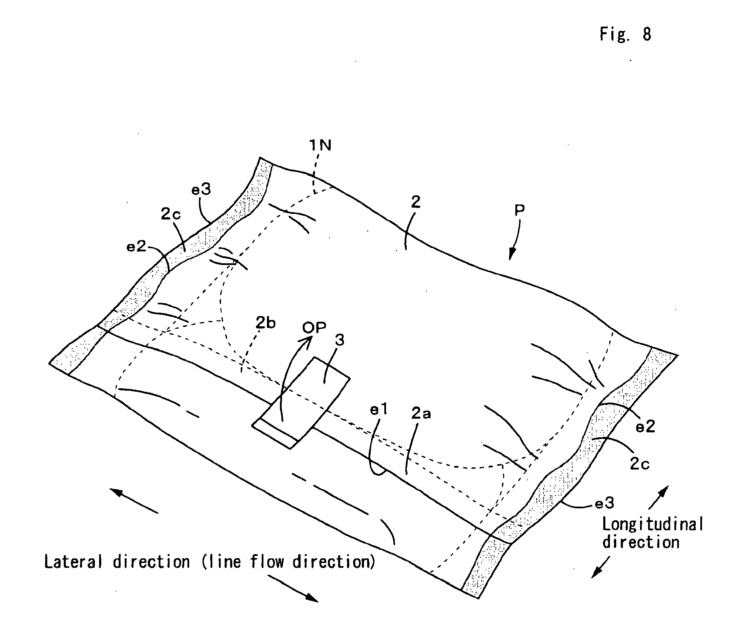

FIG. 8 shows an example P of individually packaged absorbent articles according to the present invention. This example P is structured of a sanitary napkin 1N as an absorbent article, and a package sheet 2 to pack the same made of a polyethylene sheet, a polypropylene sheet, a polyurethane sheet or the like. This example P is basically same as the conventional example shown in FIG. 1 - FIG. 4 except for the mode of its pressure sealing.

That is, the package sheet 2 is formed in a cylindrical shape such that both end parts 2a, 2b thereof in a longitudinal direction orthogonal to a packaging line flow direction are overlapped with each other, and an end edge e1 of a front surface side portion 2a of an overlapped portion serves as an opening start end, and overlapped portions 2c, 2c of both end parts in a lateral direction along the packaging line flow direction are sealed by pressure by emboss sealing or the like. And a sanitary napkin 1N is included in the package sheet 2 at its center. Recently, for compact packaging, it is general to individually package the sanitary napkin 1N in a state being folded in three as shown in the figure. However, in the present invention, the sanitary napkin 1N may be packaged in a state being folded in four, or in a state being folded in two, or it may be packaged without being folded.

Further, in order to openably seal the opening start end e1, one end of a fixing tape 3 is fixed to the opening direction side (surface side portion 2a) of the opening start end e1 in a substantially detachable manner, and the other end is adhered to the opposite side of the opening start end in a detachable manner. When both the end parts in the longitudinal direction are joined by only such a fixing tape 3, there occurs a clearance between the front surface side portion 2a and the rear surface side portion 2b at both sides of the fixing tape 3. Therefore, in a further preferred embodiment, the rear surface of the front surface side portion 2a and the rear surface side portion 2b may be adhered intermittently or continuously in the longitudinal direction in a detachable manner. In this case, its user may pinch the fixing tape 3, and pull the same to an opening direction OP, and open the package.

The present invention is characterized mainly by the mode of the pressure sealing. In the present invention, basically, the pressure sealed portions 2c, 2c are formed so as to be substantially continuous from one end to the other end in the longitudinal direction, and the end edges e2, e2 at the center side of the lateral direction of the pressure sealed portions 2c, 2c are not formed each into a straight line along the longitudinal direction. Therefore, as shown in FIG. 8 and FIG. 9, the end edges e2 at the center side in the lateral direction of the pressure sealed portions 2c, 2c may be formed into wave shapes where the top and the bottom protrude in an arc shape in the lateral direction. The following FIG. 10 - FIG. 13 show embodiments of an absorbent article which is not covered by the present invention but illustrate alternative solutions. As shown in FIG. 10, the end edges e2 at the center side in the lateral direction of the pressure sealed portions 2c, 2c may be formed into wave shapes where either the top or the bottom curves at a sharp angle. Further, as shown in FIG. 11, the end edges e2 at the center side in the lateral direction of the pressure sealed portions 2c, 2c may be formed into zigzag shapes where both of the top and the bottom curve at a sharp angle. Furthermore, though not illustrated herein, the end edges e2 at the center side in the lateral direction of the pressure sealed portions 2c, 2c may be formed into curved shapes of arc shapes or the like too. In a simpler mode, as shown in FIG. 12 and FIG. 13, the end edges e2 at the center side in the lateral direction of the pressure sealed portions 2c, 2c may be formed into straight lines inclined to the longitudinal direction.

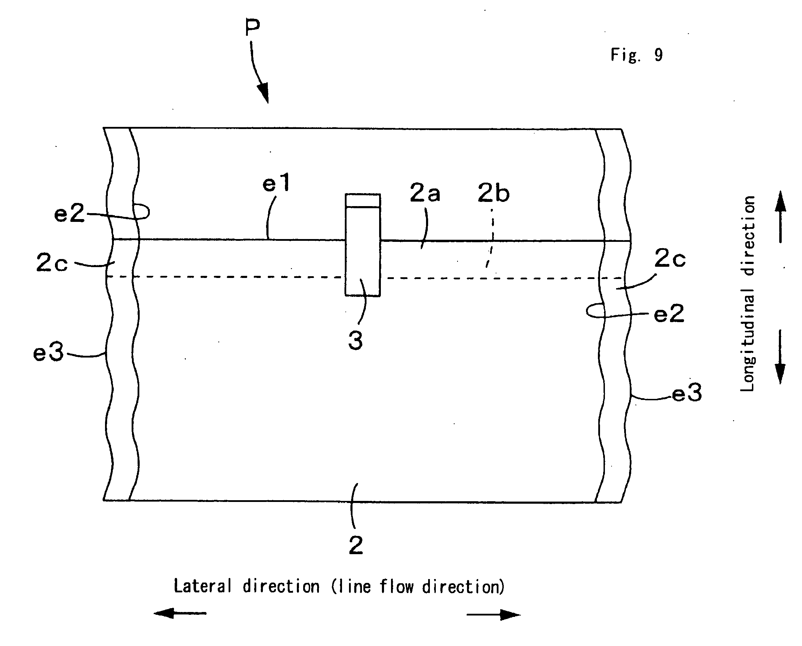

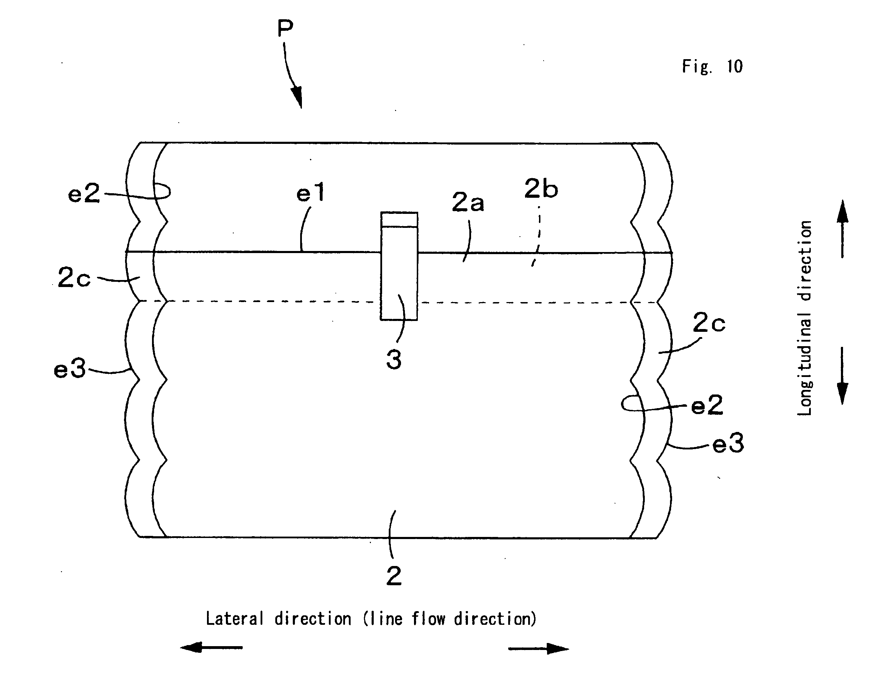

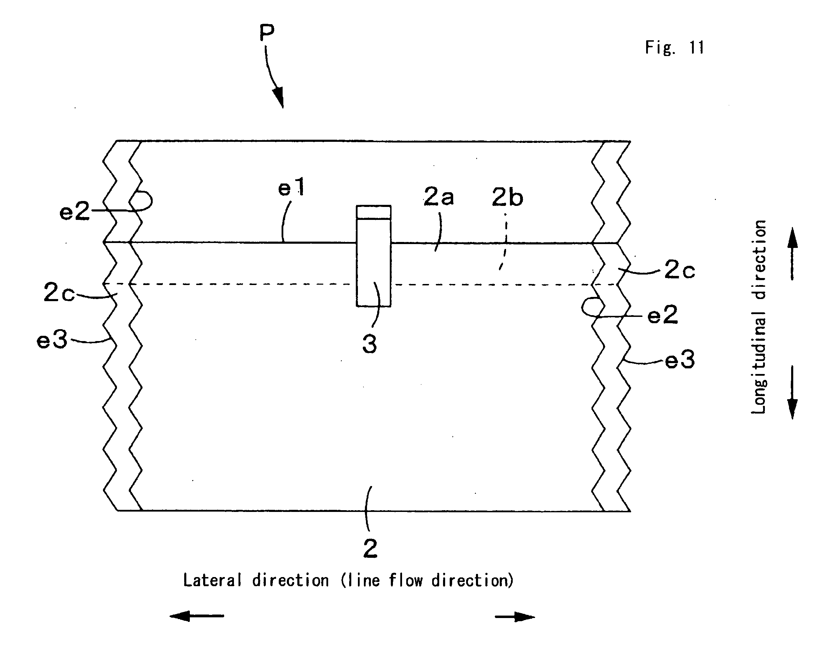

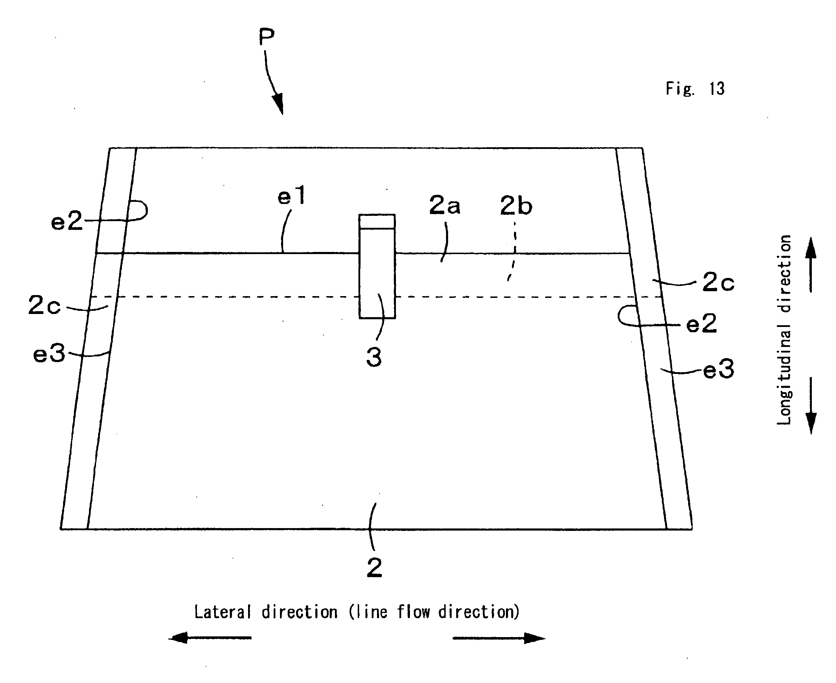

The end edges e2, e2 at the center side of the pressure sealed portions 2c may be formed to be symmetrical at the center side in the longitudinal direction, as shown in FIG. 10 and FIG. 13, or may be formed to be asymmetrical. In the latter case, the end edges e2, e2 at the center side of the pressure sealed portions 2c may be formed in parallel so as to correspond to the lateral direction as shown in FIG. 9 and FIG. 11.

In a further preferred embodiment, as shown in FIG. 9 and also in the alternative solutions shown in FIG. 10 - 12 which are not covered by the invention, the end edges e3, e3 in the lateral direction of the package sheet 2 are formed in parallel so as to laterally correspond to the end edges e2, e2 of the pressure sealed portions 2c, 2c on the lateral center side thereof. In this case, the pressure sealed portions 2c, 2c show a geometric shape with a sense of unity, and the showing such as functional beauty and premium accents, and lovely looks become remarkably preferable. Further, the pressure sealed portions 2c, 2c extend in the same width along the end edges e3, e3 in the lateral direction of the package sheet 2, and accordingly, in the pressure sealing modes as shown in FIG. 9 - FIG. 11, the detachment resistance of the pressure sealed portions 2c, 2c on opening the package does not become so unstable, and it is possible to open the package smoothly.

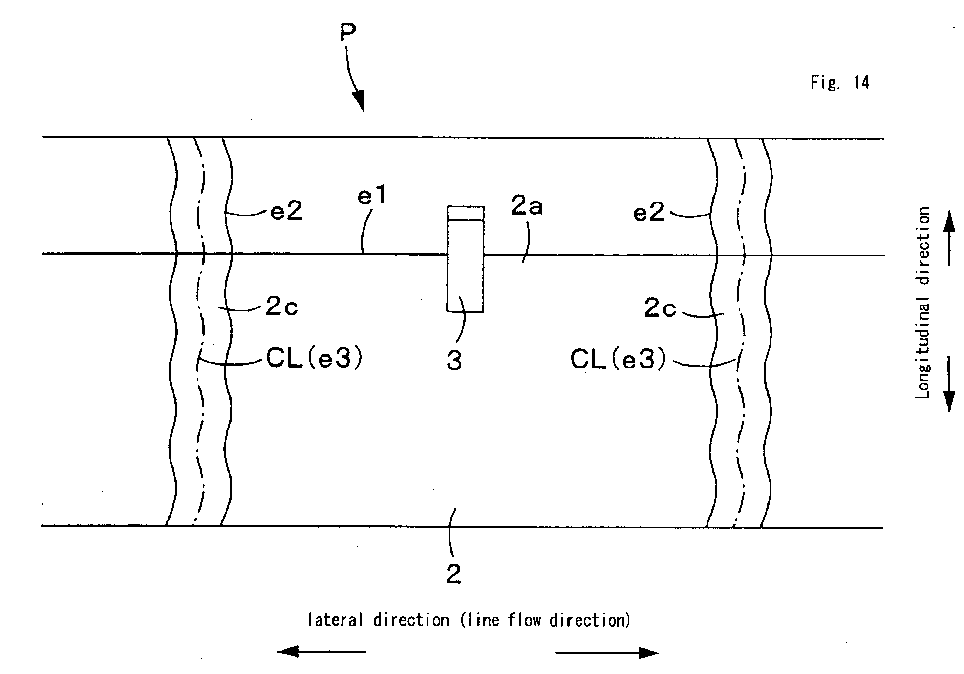

Meanwhile, in the case when the end edges e2, e2 at the center side of the pressure sealed portions 2c, 2c are formed in parallel in the lateral direction as shown in FIG. 9 and FIG. 11, as shown in FIG. 14, after carrying out pressure sealing in double width, and cutting may be made along the center line CL in the lateral direction. On the other hand, as shown in FIG. 10 and FIG. 13 for example, in the case when the end edges e2, e2 at the center side of the pressure sealed portions are formed to be symmetrical with respect to the center in the longitudinal direction, an unnecessary portion is formed at the center in the lateral direction of the pressure sealed portions, and accordingly it is necessary to carry out pressure sealing with the margin of the portion saved (not illustrated).

However, in the present invention, the end edges e3, e3 in the lateral direction of the package sheet 2 may not be formed in parallel so as to laterally correspond to the end edges e2, e2 of the pressure sealed portions 2c, 2c on the lateral center side thereof. According to an alternative solution which is not covered by the present invention from the viewpoint of easy cutting work, the end edges e3, e3 in the lateral direction of the package sheet 2 may be formed for example in a straight line along the longitudinal direction as shown in FIG. 8.

Moreover, in a further preferred embodiment, as shown in FIG. 9 and FIG. 11 - FIG. 13, a part or whole on the opening direction side of the end edges e2 of the pressure sealed portions 2c on the lateral center side thereof extends from the opening start end e1 in the opening direction so as to be away with a distance from the lateral center side. Such a mode may be applied to the pressure sealed portion 2c at one side as shown in FIG. 9, FIG. 11 and FIG. 12, or may be applied to the pressure sealed potions 2c, 2c at both the sides as shown in FIG. 13.

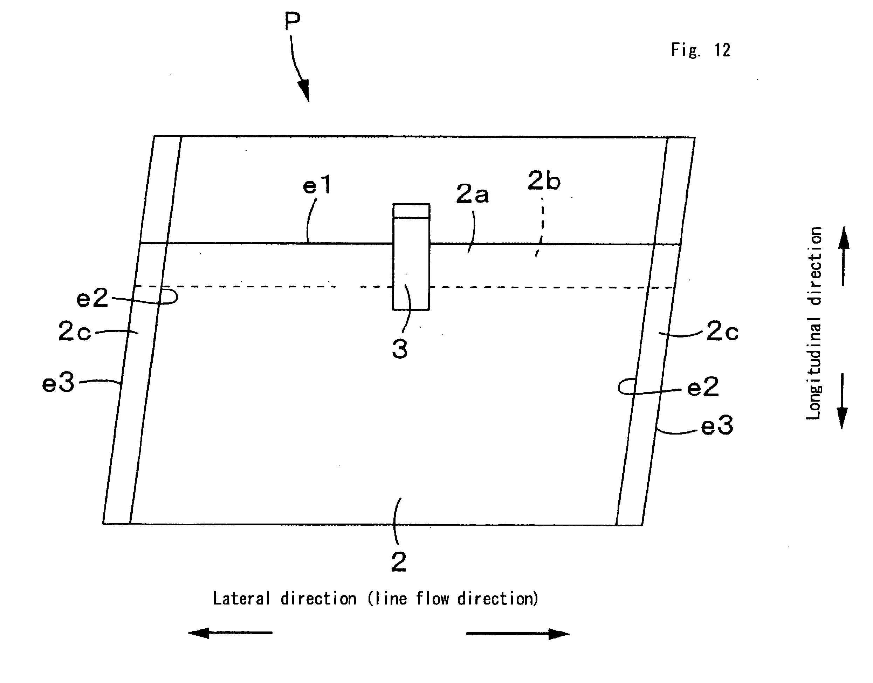

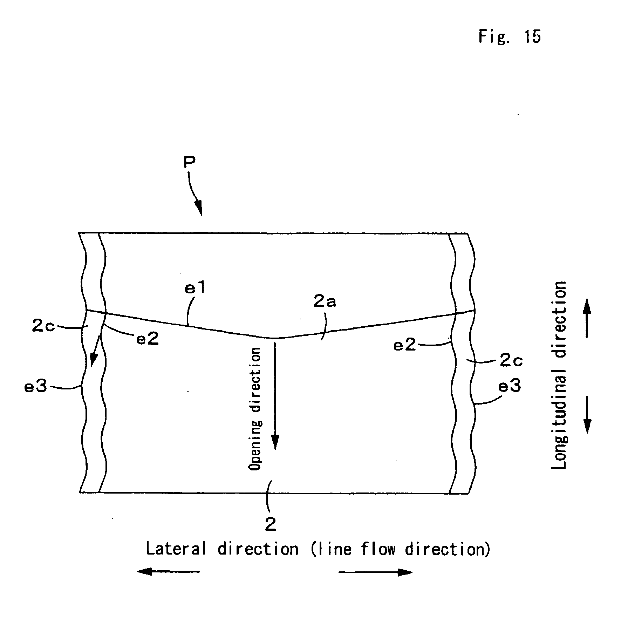

In this case, as shown by an arrow in FIG. 15, when its user pinches the fixing tape 3 (not illustrated therein), and pulls the front surface side portion 2a to the opening direction side, the sealing of the front surface side portion 2a of the package sheet 2 is released, and the center of the front surface side portion 2a is raised, then the force works from the center side of the pressure sealed portions 2c, 2c at both the sides to the diagonal direction getting away (or toward the outside), and detachment progresses. At this moment, if a part or whole on the opening direction side of the end edges of the pressure sealed portions on the lateral center side thereof extends from the opening start end e1 in the opening direction so as to be away with a distance from the lateral center side, the direction of the force that works onto the pressure sealed portions 2c, 2c becomes close to the extending direction of the end edges e2 of the pressure sealed portions 2c, 2c. Consequently, it is possible to open the package smoothly. Meanwhile, this effect is obtained at least in the beginning of opening the package in the examples shown in FIG. 9, FIG. 10 and FIG. 11, and in the examples shown in FIG. 12 and FIG. 13, it works until the package is opened completely. Further, this effect is made in the pressure sealed portion 2c at one side in FIG. 9, FIG. 11 and FIG. 12, and in the examples shown in FIG. 10 and FIG. 13, it is obtained in the pressure sealed portions 2c, 2c at both the sides. And, in any case, the advantage that opening can be started smoothly is obtained.

### <Preferred Embodiment of Manufacturing Method>

The special shapes of the end edges e2, e2 at the center side of the pressure sealed portions 2c, 2c according to the present invention are effective in packaging work, in particular, in a manufacture line. In the present invention, a method of individual packaging for forming such special sealed portions 2c, 2c is proposed. In a method of manufacturing individually packaged absorbent articles according to the present invention, the flow of packaging process itself is basically made in the same manner as in the prior art shown in for example FIG. 5, and only in pressure sealing, sealing with special shapes is carried out. That is, the manufacturing method of the present invention is based on that the pressure sealing that substantially continues from one end to the other end in the width direction of the package sheet 2 is carried out such that each of a preceding edge x1 and a following edge x2 of the convex streak 10p in a rotation direction presses at a time only a part of an entire length from one end to the other end in the width direction.

For example, in pressure sealing of a straight line inclined to the longitudinal direction as shown in FIG. 12, the pressure sealing mode shown in FIG. 7 is adopted. In this case, a pressure roll 10 where a convex streak 10p is formed along the spiral direction on external circumferential surface may be used. On the other hand, in pressure sealing of an inclined straight line as shown in FIG. 13, a pressure roll where the preceding edge and the following edge of the convex streak are inclined to the roll width direction, and the inclined direction of the preceding edge and the inclined direction of the following edge are opposite (not illustrated) is used. In the wave shaped pressure sealing 2c, 2c as shown in FIG. 9, a pressure roll (not illustrated) having a convex streak extending in wave shape along the width direction on external circumferential surface may be used.

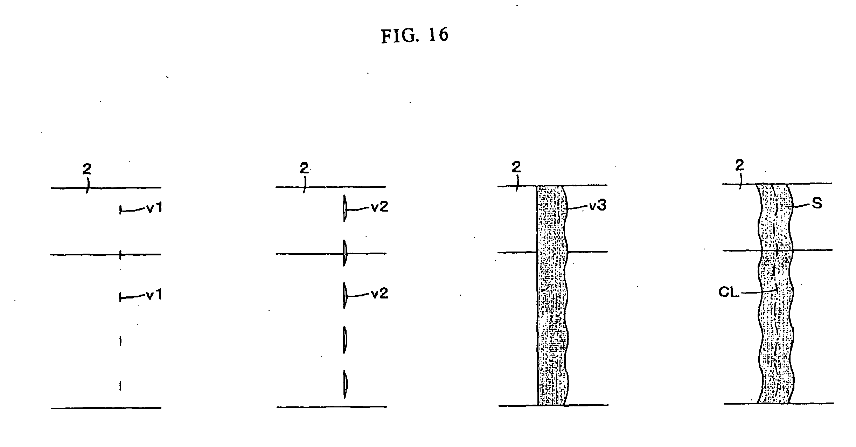

Herein, with reference to the wave shaped pressure sealing as shown in FIG. 9, advantages of the sealing modes of the present invention are explained in details once again. Now, as shown in stages in FIG. 16, suppose the state where the preceding edge of the convex streak of the roll contacts with the package sheet 2. As shown in (a) - (c) in the figure, in the convex streak, the portions v1 that first contact with the package sheet 2 in the roll width direction first shift to the surface contact state. At this moment, not only the preceding edges at the position that contact with the package sheet 2 with delay contact with the package sheet 2, but the ranges v2, v3 including the portions that first start contacting and shift to the surface contact state contact with the package sheet 2. That is, when the wave shaped pressure seal is formed, at the moment of contact start, as shown as (a) in the figure, intermittent pressing in the width direction is carried out, and then as shown as (b) and (c) in the figure, the area is expanded in sequence from the contact start position v1 as the start point not only in the width direction but also in the line flow direction. When the following edges of the convex streaks leave the package sheet 2, the above contact state is reversed. Accordingly, even if cutting occurs due to the preceding and following edges of the convex streak, cutting just appears intermittently in the longitudinal direction of the package sheet 2, and it is possible to prevent a situation where the entire end edges e2 at the center side of the lateral direction of the pressure sealed portion S are cut.

Then, after the pressure seal S is carried out in the manner mentioned above, an appropriate position (normally the center) in the line flow direction of the pressure sealed portion S is cut by cutting apparatus 11 such as roll cutter apparatus or the like, and thereby individually packaged absorbent articles are manufactured. Meanwhile, other points than pressure seal have been explained in the prior art section, therefore, detailed explanations thereof are omitted here.

### [Industrial Applicability]

The present invention may be applied to, in addition to sanitary napkins, already known absorbent articles such as disposable paper diapers and the like, and it is suitable for absorbent articles that need to be hygienic.

### [Brief Description of Drawings]

FIG. 1 is a top view showing an example according to the prior art.

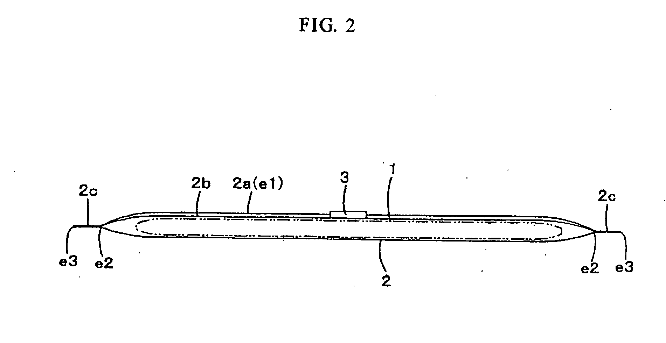

FIG. 2 is a cross sectional view at II-II in FIG. 1.

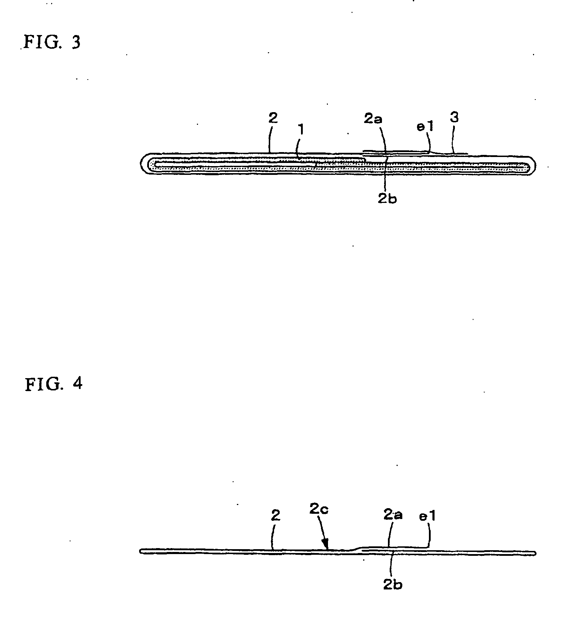

FIG. 3 is a cross sectional view at III-III in FIG. 1.
FIG. 4 is a cross sectional view at IV-IV in FIG. 1.
FIG. 5 is a flow chart of a packaging process.
FIG. 6 is a comparative figure consisting of cross sectional views and top views showing the state transition of a pressure seal according to the prior art.
FIG. 7 is a comparative figure consisting of cross sectional views and top views showing the state transition of a pressure seal according to the present invention.
FIG. 8 is a perspective view showing an individually packaged absorbent article according to the present invention.
FIG. 9 is a top view showing an individually packaged absorbent article according to the present invention.
FIG. 10 is a top view showing another example according to the background art.
FIG. 11 is a top view showing still another example according to the background art.
FIG. 12 is a top view showing further another example according to the background art.
FIG. 13 is a top view showing still further another example according to the background art.
FIG. 14 is a top view showing an example of cut edges.
FIG. 15 is a top view for explaining the working direction of opening force and the like.
FIG. 16 is a schematic view showing the state transition of a pressure seal according to the present invention.

### [Description of Codes]

- 1: Absorbent article
- 2: Package sheet
- 3: Fixing tape
- e1: Unpacking paper end
- e2: End edge at the center side of the lateral direction
- e3: End edge of package sheet

### Drawings

FIG. 1
   1 Lateral direction (line flow direction)
   2 Longitudinal direction Lateral
FIG. 5
   1 Width direction
   2 Absorbent article
   3 Fixing tape
FIG. 8
   1 Lateral direction (line flow direction)
   2 Longitudinal direction
FIG. 9
   1 Lateral direction (line flow direction)
   2 Longitudinal direction
FIG. 10
   1 Lateral direction (line flow direction)
   2 Longitudinal direction
FIG. 11
   1 Lateral direction (line flow direction)
   2 Longitudinal direction
FIG. 12
   1 Lateral direction (line flow direction)
   2 Longitudinal direction
FIG. 13
   1 Lateral direction (line flow direction)
   2 Longitudinal direction
FIG. 14
   1 Lateral direction (line flow direction)
   2 Longitudinal direction
FIG. 15
   1 Opening direction
   2 Lateral direction (line flow direction)
   3 Longitudinal direction

## Claims

1. An individually packaged absorbent article comprising an absorbent article and a package sheet (2) to pack the absorbent article, wherein
the package sheet is formed in a cylindrical shape such that both end parts (2a, 2b) thereof In a longitudinal direction are overlapped with each other, and an end edge (e1) of a front surface side portion (2a) of an overlapped portion serves as an opening start end, and overlapped portions of both end parts (2a, 2b) in a lateral direction are sealed by pressure, and wherein
the pressure sealed portions (2c) are formed so as to substantially continue from one end to the other end in the longitudinal direction, and end edges (e2) of the pressure sealed portions (2c)on lateral center side are not formed to be straight along the longitudinal direction wherein the end edges (e2) at the center side in the lateral direction of the pressure sealed portions (2c) are formed into wave shapes where the top and the bottom protrude in an arc shape in the lateral direction.

2. The individually packaged absorbent article according to claim 1, wherein the end edges (2a, 2b) in the lateral direction of the package sheet (2) are formed in parallel so as to laterally correspond to the end edges (e2) of the pressure sealed portions (2c) on the lateral center side thereof.

3. The individually packaged absorbent article according claim 1 or 2, wherein a part or whole on the opening direction side of the end edges (e2) of the pressure sealed portions (2c) on the lateral center side thereof extends from the opening start end in the opening direction so as to be away with a distance from the lateral center side.

4. A method of manufacturing the individually packaged absorbent article according to claim 1, comprising steps of
feeding a band shaped package sheet (2) to a packaging line;
placing absorbent articles on the band shaped package sheet (2);
folding back an end part on one side and an end part on the other side in a width direction of the band shaped package sheet (2) and thereby packing the absorbent articles;
passing the band shaped package sheet (2) containing the absorbent articles between a pair of rolls (10) each having a convex streak (10p) that substantially continues from one end to the other end in the width direction, and pressing a portion to be sealed preceding and a portion to be sealed following in a flow direction with respect to a part of the band shaped package sheet (2) which contains the absorbent article between the convex streak (10p) of the roll (10) at one side and the convex streak (10p) of the roll (10) at the other side, and thereby carrying out a pressure sealing that substantially continues from one end to the other end in the width direction, and cutting each of the pressure sealed portions(2c) at its middle in the line flow direction, and thereby obtaining individually packaged absorbent articles; wherein
the pressure sealing that substantially continues from one end to the other end in the width direction of the package sheet (2) is carried out such that each of a preceding edge (x1) and a following edge (x2) of the convex streak (10p) in a rotation direction presses at a time only a part of an entire length from one end to the other end in the width direction.

## Patentansprüche

1. Einzeln verpackter Absorptionsartikel, der einen Absorptionsartikel und eine Verpackungsfolie zum Verpacken des Absorptionsartikels umfasst, wobei
die Verpackungsfolie in eine zylindrische Form geformt ist, so dass beide Endteile (2a, 2b) hiervon in einer Längsrichtung miteinander überlappen und eine Endkante (e1) eines Seitenabschnitts (2a) einer vorderen Oberfläche eines überlappenden Abschnitts als ein Öffnungsanfangsende dient und überlappte Abschnitte beider Endteile (2a, 2b) in einer seitlichen Richtung durch Druck versiegelt sind, wobei
die durch Druck versiegelten Abschnitte (2c) so geformt sind, dass sie von einem Ende zum anderen Ende in der Längsrichtung im Wesentlichen ununterbrochen sind und Stirnkanten (e2) der durch Druck versiegelten Abschnitte (2c) auf Seiten einer seitlichen Mitte nicht geradlinig längs der Längsrichtung geformt sind, wobei die Stirnkanten (e2) auf Seiten der Mitte in der seitlichen Richtung der durch Druck versiegelten Abschnitte (2c) wellenförmig ausgebildet sind, wobei die Oberseite und die Unterseite in seitlicher Richtung bogenförmig vorstehen.

2. Einzeln verpackter Absorptionsartikel nach Anspruch 1, wobei die Stirnkanten (2a, 2b) in der seitlichen Richtung der Verpackungsfolie (2) parallel geformt sind, so dass sie seitlich den Stirnkanten (e2) der durch Druck versiegelten Abschnitte (2c) auf Seiten der seitlichen Mitte hiervon entsprechen.

3. Einzeln verpackter Absorptionsartikel nach Anspruch 1 oder 2, wobei sich ein Teil der oder die gesamte Öffnungsrichtungsseite der Stirnkanten (e2) der durch Druck versiegelten Abschnitte (2c) auf Seiten der seitlichen Mitte von dem Öffnungsanfangsende in der Öffnungsrichtung erstreckt, damit er sich in einem Abstand von der Seite der seitlichen Mitte befindet.

4. Verfahren zum Herstellen des einzeln verpackten Absorptionsartikels nach Anspruch 1, das die folgenden Schritte umfasst:
Zuführen einer bandförmigen Verpackungsfolie (2) zu einer Verpackungslinie;
Anordnen von Absorptionsartikeln auf der bandförmigen Verpackungsfolie (2);
Zurückfalten eines Endteils auf einer Seite und eines Endteils auf der anderen Seite in einer Breitenrichtung der bandförmigen Verpackungsfolie (2) und **dadurch** Verpacken der Absorptionsartikel;
Bewegen der bandförmigen Verpackungsfolie (2), die die Absorptionsartikel enthält, zwischen einem Paar von Rollen (10), wovon jede einen konvexen Streifen (10p) besitzt, der von einem Ende zum anderen Ende in der Breitenrichtung im Wesentlichen ununterbrochen ist und einen vorhergehenden zu versiegelnden Abschnitt und einen nachfolgenden zu versiegelnden Abschnitt in einer Flussrichtung in Bezug auf einen Teil der bandförmigen Verpackungsfolie (2), die den Absorptionsartikel enthält, zwischen dem konvexen Streifen (10p) der Rolle (10) auf einer Seite und dem konvexen Streifen (10p) der Rolle (10) auf der anderen Seite zu pressen, um **dadurch** eine Druckversiegelung auszuführen, die von einem Ende zum anderen Ende in der Breitenrichtung im Wesentlichen ununterbrochen ist, und Durchschneiden jedes der durch Druck versiegelten Abschnitte (2c) in ihrer Mitte in der Linienflussrichtung, um **dadurch** einzeln verpackte Absorptionsartikel zu erhalten; wobei
die Druckversiegelung, die von einem Ende zum anderen Ende in der Breitenrichtung der Verpackungsfolie (2) im Wesentlichen ununterbrochen ist, in der Weise ausgeführt wird, dass sowohl eine vorhergehende Kante (x1) als auch eine nachfolgende Kante (x2) des konvexen Streifens (10p) in einer Drehrichtung zu einer Zeit nur einen Teil einer gesamten Länge von einem Ende zum anderen Ende in der Breitenrichtung presst.

## Revendications

1. Article absorbant emballé individuellement comprenant un article absorbant et une feuille d'emballage (2) pour emballer l'article absorbant, la feuille d'emballage étant façonnée en une forme cylindrique de manière que ses deux parties d'extrémité (2a, 2b), dans une direction longitudinale, se chevauchent, et un bord d'extrémité (e1) d'une partie latérale de surface avant (2a) d'une partie en chevauchement serve d'extrémité de début d'ouverture, et les parties en chevauchement des deux parties d'extrémité (2a, 2b) dans une direction transversale sont soudées par pression, et
les parties soudées par pression (2c) étant formées de manière à se prolonger sensiblement d'une extrémité à l'autre extrémité dans la direction longitudinale, et des bords d'extrémité (e2) des parties soudées par pression (2c) sur le côté central transversal ne sont pas formés pour être rectilignes dans la direction longitudinale, les bords d'extrémité (e2) sur le côté central dans la direction transversale des parties soudées par pression (2c) étant façonnés en des ondulations dont le sommet et la base font saillie en une forme arquée dans la direction transversale.

2. Article absorbant emballé individuellement selon la revendication 1, dans lequel les bords d'extrémité (2a, 2b) dans la direction transversale de la feuille d'emballage (2) sont formés en parallèle de manière à correspondre transversalement aux bords d'extrémité (e2) des parties soudées par pression (2c) sur leur côté central transversal.

3. Article absorbant emballé individuellement selon la revendication 1 ou 2, dans lequel une portion ou la totalité, sur le côté de direction d'ouverture, des bords d'extrémité (e2) des parties soudées par pression (2c) sur leur côté central transversal s'étend depuis l'extrémité de début d'ouverture dans la direction d'ouverture de façon à être éloignée d'une certaine distance du côté central transversal.

4. Procédé de fabrication de l'article absorbant emballé individuellement selon la revendication 1, comprenant les étapes consistant à :
distribuer une feuille d'emballage (2) en forme de bande jusqu'à une chaîne d'emballage ;
placer des articles absorbants sur la feuille d'emballage en forme de bande (2) ;
replier une partie d'extrémité sur un côté et une partie d'extrémité sur l'autre côté dans le sens de la largeur de la feuille d'emballage en forme de bande (2) et emballer ainsi les articles absorbants ;
faire passer la feuille d'emballage en forme de bande (2) contenant les articles absorbants entre une paire de rouleaux (10) comportant chacun une strie convexe (10p) qui se prolonge sensiblement d'une extrémité à l'autre dans le sens de la largeur et presser une partie à souder précédente et une partie à souder suivante dans une direction de transport, par rapport à une partie de la feuille d'emballage en forme de bande (2) qui contient l'article absorbant, entre la strie convexe (10p) du rouleau (10) sur un côté et la strie convexe (10p) du rouleau (10) sur l'autre côté, et réaliser ainsi un soudage par pression qui se prolonge sensiblement d'une extrémité à l'autre dans le sens de la largeur, et découper chacune des parties soudées par pression (2c) en son milieu dans la direction de transport sur la chaîne, et obtenir ainsi des articles absorbants emballés individuellement ; le soudage par pression qui se prolonge sensiblement d'une extrémité à l'autre dans le sens de la largeur de la feuille d'emballage (2) étant réalisé de manière que chaque bord parmi un bord précédent (x1) et un bord suivant (x2) de la strie convexe (10p) dans le sens de rotation presse à la fois uniquement une partie d'une longueur entière d'une extrémité à l'autre dans le sens de la largeur.
